# EUROPEAN PATENT APPLICATION

(11) **EP 4 609 803 A1**
(43) Date of publication of application: **03.09.2025**
(21) Application number: 24876733.7
(22) Date of filing: 07.11.2024
(51) Int. Cl.: A61B 17/072

(54) **SURGICAL INSTRUMENT AND CONTROL METHOD THEREFOR**

(30) Priority: 11.10.2023 CN 202311318090
(71) Applicant: Hangzhou Mindray Medical Technology Co., Ltd., Hangzhou, Zhejiang 311508 (CN)
(72) Inventor: LI, Mouyuan, Hangzhou, Zhejiang 311508 (CN); DAI, Jian, Hangzhou, Zhejiang 311508 (CN); ZHENG, Han, Hangzhou, Zhejiang 311508 (CN); ZUO, Pengfei, Hangzhou, Zhejiang 311508 (CN)
(74) Representative: KIPA AB
(86) International application number: PCT/CN2024/130500
(87) International publication number: WO 2025/077928

(57) **Abstract**

A surgical instrument and a control method thereof are disclosed. The surgical instrument includes a handle assembly; a barrel assembly including a transmission mechanism; an effector assembly including a first jaw and a second jaw for clamping a tissue; a staple cartridge assembly including a firing member connected with a transmission mechanism; a drive source capable of driving, via the transmission mechanism, the firing member to move, wherein the firing member fires one or more stitching staples to stitch the tissue while moving; a controller for at least determining a movement rate of the firing member when firing the stitching staple according to a time parameter related to squeezing the tissue when the first jaw and the second jaw are closed. Through the above surgical instrument and their control methods, the yield of staple is improved to better stitch the tissue.

## Description

### TECHNICAL FIELD

The disclosure relates to a technical field of medical instrument, and more particularly to a surgical instrument and control method thereof.

### BACKGROUND

At present, a minimally invasive surgery occupies an important position in surgeries, and a surgical stapling instrument is a commonly used surgical instrument in a minimally invasive surgery. A working principle of the surgical stapling instrument is to clamp a jaw of an end effector at a designated position near a lesion site, excise a tissue through a cutting knife of the end effector, and stitch through a staple cartridge assembly of the end effector while excising.

The principle of the staple cartridge assembly for stitching is similar to that of a stapler for binding papers. The staple cartridge assembly is mainly composed of a stitching staple, a staple anvil, a staple cartridge and other assemblies. The surgical stapling instrument fires the stitching staple through a firing member to eject the stitching staple, so as to stitch a tissue. How to better cut/stitch a tissue in clinic, especially cut/stitch a thick tissues, is one of problems to be improved or solved.

### SUMMARY

According to a first aspect, some embodiments provide a surgical instrument, including:
a handle assembly;
a barrel assembly, wherein a proximal end of the barrel assembly is connected with the handle assembly, and the barrel assembly includes a transmission mechanism;
an end effector assembly, wherein a distal end of the barrel assembly is connected with the end effector assembly, and the end effector assembly includes a first jaw and a second jaw for clamping a tissue;
a staple cartridge assembly, which is arranged between the first jaw and the second jaw, and includes a firing member which is connected with the transmission mechanism;
a drive source, which is capable of driving, via the transmission mechanism, the firing member to move, wherein the firing member is configured to fire one or more stitching staples to stitch the tissue while moving;
a controller, which is at least configured to determine a movement rate of the firing member when the firing member fires the stitching staple(s), according to a time parameter which is related to squeezing the tissue when the first jaw and the second jaw are closed, so as to control the firing member to fire the stitching staple(s) at the movement rate determined.

According to a second aspect, some embodiments provide a control method for a surgical instrument, wherein the surgical instrument includes:
a handle assembly;
a barrel assembly, wherein a proximal end of the barrel assembly is connected with the handle assembly, and the barrel assembly includes a transmission mechanism;
an end effector assembly, wherein a distal end of the barrel assembly is connected with the end effector assembly, and the end effector assembly includes a first jaw and a second jaw for clamping a tissue;
a staple cartridge assembly, which is arranged between the first jaw and the second jaw, and includes a firing member which is connected with the transmission mechanism;
a drive source, which is capable of driving, via the transmission mechanism, the firing member to move, wherein the firing member is configured to fire one or more stitching staples to stitch the tissue while moving;
wherein the method includes:
   at least determining a movement rate of the firing member when the firing member fires the stitching staple(s), according to a time parameter which is related to squeezing the tissue when the first jaw and the second jaw are closed, so as to control the firing member to fire the stitching staple(s) at the movement rate determined.

According to a third aspect, some embodiments provide a computer-readable storage medium on which a program is stored, wherein the program is capable of being executed by a processor, so as to implement the above method.

According to the surgical instrument and a control method for a surgical instrument of above embodiments, the movement rate of the firing member when the firing member fires the stitching staple is determined according to the time parameter which is related to the squeezing of the tissue when the first jaw and the second jaw are closed, such that a better yield of stitching staple and a better stitch effect can be achieved.

### BRIEF DESCRIPTION OF THE DRAWINGS

FIG. 1 is a structural diagram of a surgical instrument of an embodiment.
FIG. 2 is a structural diagram of an end effector assembly of an embodiment.
FIG. 3 is a structural diagram of a staple cartridge assembly of an embodiment.
FIG. 4 is a structural diagram of a staple cartridge assembly of another embodiment.
FIG. 5 is a flowchart of a control method for a surgical instrument according to an embodiment.
FIG. 6 is a flowchart of a control method for a surgical instrument according to another embodiment.
FIG. 7 is a flowchart of a control method for a surgical instrument according to another embodiment.
FIG. 8 is a flowchart of a control method for a surgical instrument according to another embodiment.

100. Handle assembly; 110. Fixed handle; 120. Movable handle;
200. Barrel assembly; 210. Transmission mechanism;
300. End effector assembly; 310. First jaw; 320. Second jaw;
400. Staple cartridge assembly; 410. Mounting base;
412. Tissue junction surface; 413. Movable cavity; 414. Support portion; 415. Position-limiting portion;
420. Firing member; 430. Cutting knife.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described so as to make this disclosure better understood. However, one skilled in the art can easily recognize that some of the features can be omitted in different cases, or can be replaced by other elements, materials and methods. In some cases, some operations related to this disclosure are not shown or described in the description, so as to avoid a core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in this disclosure may be combined in any appropriate manner to form various embodiments. At the same time, the steps or actions in the method description can also be exchanged or regulated in order in a manner obvious to one skilled in the art. Therefore, the various sequences in the description and the drawings are only for the purpose of clearly describing a certain embodiment, and do not mean that they are necessary sequences, unless it is otherwise specified that one of the sequences must be followed.

The terms "first", "second", etc. in the description are used to distinguish different objects, rather than to describe a specific order. In addition, the terms "connection", and "linkage" mentioned in this disclosure include direct and indirect connection (linkage), unless otherwise specified.

The inventor found that although adjusting a firing rate while firing can improve a yield of staple for a thick tissue, the adjustment for the firing rate, when the tissue is squeezed in place, has a limited effect on the yield of staple for the thick tissue. Therefore, a most important idea of this disclosure is to take a time parameter which is related to squeezing the tissue as one of the basis for determining a movement rate of the firing member. Specifically, the inventor learned that under a condition of a constant squeezing force, the tissue will denature with a squeezing time until the tissue is in equilibrium, that is, a creep property of the tissue. In addition, under an action of an external force, when the external force remains constant, a stress of the tissue decreases with time until equilibrium. When the external force disappears, the tissue tends to return to its original state. Therefore, the shorter the squeezing time, the greater the internal stress in the tissue and the smaller the deformation, accordingly, it is necessary to reduce the firing rate and extend the squeezing time, based on setting an initial rate of the firing member to be in a positive correlation with a pre-squeezing time and a squeezing time.

"Distal end" in this disclosure refers to an end which is away from an operator when said operator operates the surgical instrument, and a "proximal end" refers to the end which is adjacent to an operator when said operator operates the surgical instrument. "Axial" in this disclosure refers to a direction of a connection line between a center of the distal end and a center of the proximal end, and "radial" refers to a direction which is perpendicular to the axial direction.

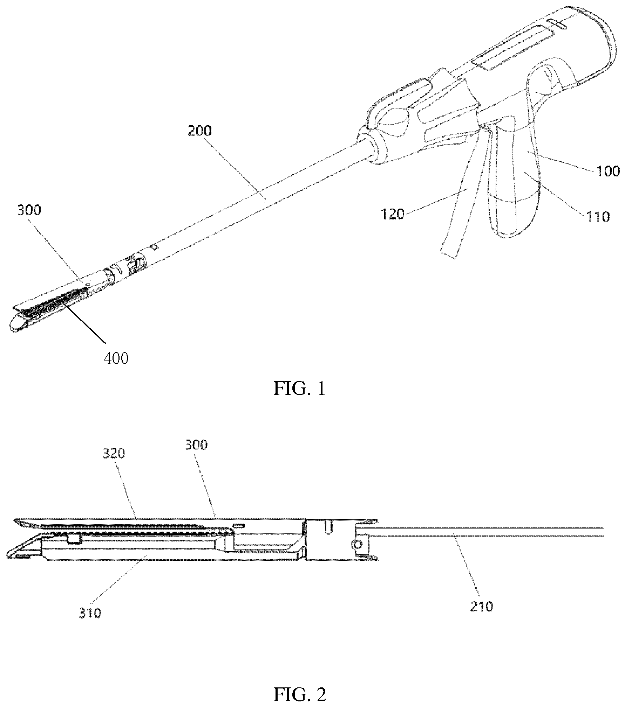

Please refer to embodiments shown in FIGS. 1 to 4, which provide a surgical instrument, including a handle assembly 100, a barrel assembly 200, an end effector assembly 300, a staple cartridge assembly 400, a drive source (unshown) and a controller (unshown). For example, the surgical instrument may be a surgical stapling instrument.

The handle assembly 100 includes a fixed handle 110 and at least one movable handle 120 for an operator to hold. A proximal end of the barrel assembly 200 is connected with the handle assembly, and a distal end of the barrel assembly 200 is connected with the end effector assembly 300. The barrel assembly 200 includes a closed transmission chain and a firing transmission chain. When operating the surgical instrument, the operator can hold the fixed handle 110 and insert the end effector assembly 300 into a lesion in a human body through the barrel assembly 200 which is slender, and then trigger the closed transmission chain through the movable handle 120 to close jaws of the end effector assembly 300 and clamp a position which is adjacent the lesion. Then the operator can trigger the firing transmission chain through the movable handle 120, so as to cut the tissue through a cutting knife 430 inside the end effector assembly 300, and to stitch the tissue through a stitching staple of the staple cartridge assembly 400 while cutting. In this embodiment, the firing transmission chain includes a transmission mechanism 210, which is configured to realize the firing of the stitching staple and the cutting of the cutting knife under an action of the drive source.

The end effector assembly 300 includes a first jaw 310 and a second jaw 320 for clamping the tissue. The first jaw 310 is movably connected with the second jaw 320, and the first jaw 310 and the second jaw 320 can move relatively to each other, so as to achieve closing and opening. A mounting cavity is formed between the first jaw 310 and the second jaw 320, the staple cartridge assembly 400 is mounted inside the mounting cavity, and the distal end of the transmission mechanism 210 extends into the mounting cavity.

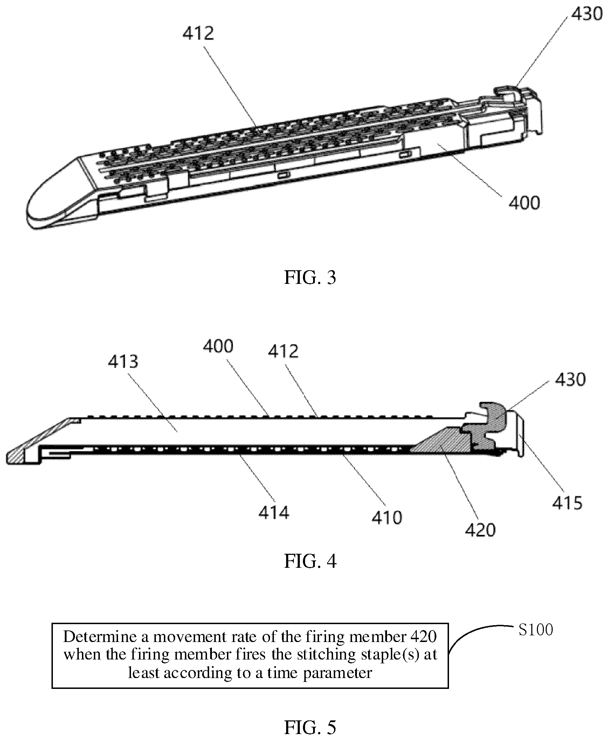

Please continue to refer to FIGS. 3 and 4. The staple cartridge assembly 400 includes a mounting base 410, a firing member 420 and a cutting knife 430. The mounting base 410 is provided with multiple mounting positions for mounting stitching staples. The mounting base 410 is provided with a tissue junction surface 412 for contacting with the tissue. The firing member 420 is movably arranged at the mounting base 410, the cutting knife 430 is movably connected with the mounting base 410, and the firing member 420 and the cutting knife 430 can move between a proximal end and a distal end of the mounting base 410. The transmission mechanism 210 can drive the firing member 420 and the cutting knife 430 to move between the proximal end and the distal end of the mounting base 410. It should be noted that the tissue junction surface 412 of the mounting base 410 refers to a surface on the mounting base 410 that contacts with a clamped tissue when clamping the tissue. It can be understood that while stitching the tissue, the stitching staple is pushed out from the tissue junction surface 412 of the mounting base 410, so as to stitching the tissue. When the stitching staple is pushed out, it is regarded as be fired by the firing member 420.

In some embodiments, the mounting base 410 is provided with a movable cavity 413 which is arranged along an axial direction of the mounting base 410; wherein the firing member 420 and the cutting knife 430 are movably arranged inside the movable cavity 413. Respective movement directions of the firing member 420 and the cutting knife 430 are defined by the movable cavity 413 to ensure that the firing member 420 and the cutting knife 430 can move between the proximal end and the distal end of the mounting base 410 along the axial direction of the mounting base.

In some embodiments, the mounting base 410 is provided with a support portion 414, which is located at a side of the movable cavity 413 which side is away from a mounting position, so as to support the firing member 420 and the cutting knife 430. The support portion 414 prevents the firing member 420 and the cutting knife 430 from being separated from the movable cavity 413 from the side of the mounting base 410 which side is away from the mounting position.

In some embodiments, the proximal end of the mounting base 410 is provided with a position-limiting portion 415, which is configured to prevent the firing member 420 and the cutting knife 430 from being separated from the movable cavity 413 from the proximal end of the mounting base 410. The position-limiting portion 415 prevents the firing member 420 and the cutting knife 430 from being separated from the movable cavity 413 from the proximal end of the mounting base 410.

In some embodiments, the staple cartridge assembly 400 may be regarded as a part of the end effector assembly 300. For example, the staple cartridge assembly 400 may be fixedly connected with the first jaw 310 or the second jaw 320 or may form an integral structure with the first jaw 310 or the second jaw 320. The staple cartridge assembly 400 can also be regarded as an assembly which is independent of the end effector assembly 300. For example, the staple cartridge assembly 400 can be detachably mounted at the first jaw 310 or the second jaw 320, so as to enable the staple cartridge assembly 400 to be regarded as a consumable which is applied to the end effector assembly 300.

The drive source is capable of driving, via the transmission mechanism 210, the firing member 420 to move. Specifically, the drive source can apply a force along an axial direction of the transmission mechanism 210 to move the transmission mechanism 210 towards a distal end, so as to enable the transmission mechanism 210 to push the firing member 420 to move. The firing member 420 fires one or more stitching staples to stitch the tissue while moving. It can be understood that when the drive source drives the transmission mechanism 210 with different drive parameters, rates of the transmission mechanism 210 are different, so that movement rates of the firing member 420 and firing rates of the stitching staple are also respectively different. In some embodiments, if the drive source is an electrical motor or a motor, a rate of the transmission mechanism 210 can be controlled by setting a current, a rotation rate, and other parameters of the motor.

In this embodiment, a start position and an end position can be defined in a stroke of the firing member 420 for moving from a proximal end to a distal end, and a process between the start position and the end position is defined as a firing process (also referred as while the firing member 420 fires). A rate for moving from the start position to the end position represents a firing rate. The start position refers to a position where the firing member 420 starts firing the stitching staple(s) while moving, and the end position refers to a position where the firing member 420 is located when a tissue stitching has been completed. In some embodiments, a position, where the firing member 420 fires part or all of the stitching staples in the mounting base 410, is defined as the end position. In other embodiments, the end position is defined by a distance, and a preset distance exists between the end position and the start position. It can be understood that the faster the firing member 420 moves from the start position to the end position, the faster the firing process ends, the faster the firing rate of the whole process, and the faster the stitching rate of the tissue. On the contrary, the slower the firing member 420 moves from the start position to the end position, the slower the firing process ends, the slower the firing rate of the whole process, and the slower the stitching rate of the tissue. The key idea of this embodiment is how to control a movement rate of the firing member 420 from the start position to the end position. It can be understood that this is also equivalent to how to control the firing rate of the stitching staple, or how to control the drive parameter of the drive source, or how to control the stitching rate of the tissue.

The controller is at least configured to determine a movement rate of the firing member 420 when the firing member fires the stitching staple(s), according to a time parameter which is related to squeezing the tissue when the first jaw 310 and the second jaw 320 are closed, so as to control the firing member 420 to fire the stitching staple(s) at the movement rate determined. It can be understood that the controller controls the movement rate of the transmission mechanism 210 by controlling the drive parameter of the drive source, so as to control the movement rate of the firing member 420. In this disclosure, the controller controls the movement rate of the firing member 420 in two dimensions according to the time parameter. The movement rate in a first dimension is the movement rate when the firing member 420 starts firing the stitching staple, that is, an initial movement rate of the firing member 420 at a start position. The movement rate in the second dimension is the movement rate of the firing member 420 while the firing member 420 fires the stitching staple. That is to say, the controller can continuously adjust the movement rate of the firing member 420 from the start position to the end position, which will be explained separately below.

In some embodiments, the surgical instrument also includes a timing apparatus for obtaining the time parameter. The timing apparatus is configured to start timing after the first jaw 310 and the second jaw 320 are closed to a designated relative position, so as to obtain the time parameter. In some embodiments, the time parameter includes a pre-squeezing time, which is from a time point when the first jaw 310 and the second jaw 320 are closed to a designated relative position to a time point when the firing member 420 starts firing the stitching staple. The controller determines the movement rate of the firing member 420 when the firing member 420 starts firing the stitching staple(s) according to the pre-squeezing time. The designated relative position can be customized a user according to a situation, or can also be a relative position that satisfies a preset condition, including but not limited to following positions.

The designated relative position can be a position where the first jaw 310 and the second jaw 320 start to clamp the tissue. For example, both the first jaw 310 and the second jaw 320 are respectively provided with a pressure sensor. When a certain value appears on both pressure sensors, it means that both the first jaw 310 and the second jaw 320 are in contact with the tissue, and then the timing apparatus obtains the time parameter through timing. It is understood that whether the first jaw 310 and the second jaw 320 start to clamp the tissue can also be determined by other means.

The designated relative position can be a designated position to where the first jaw 310 and the second jaw 320 clamp the tissue. For example, the first jaw 310 and the second jaw 320 are respectively provided with a pressure sensor. When a measurement result of the pressure sensor exceeds a preset pressure threshold, it means that the first jaw 310 and the second jaw 320 clamp the tissue to a designated position, and then the timing apparatus obtains the time parameter through timing. It is understood that whether the first jaw 310 and the second jaw 320 clamp the tissue to a designated position can also be determined by other ways.

The designated relative position can be a position at which the first jaw 310 and the second jaw 320 are closed to a preset distance from each other, or the first jaw 310 and the second jaw 320 are closed to a preset angle. For example, a distance sensor is arranged between the first jaw 310 and the second jaw 320. When the first jaw 310 and the second jaw 320 are closed to a preset distance from each other, the time parameter is obtained by timing after that. For another example, a movable connection position of the first jaw 310 and the second jaw 320 is provided with an angle measurement apparatus. When the first jaw 310 and the second jaw 320 are closed to a preset angle, the timing apparatus obtains the time parameter through timing. Even in some embodiments, a position, where the first jaw 310 and the second jaw 320 start to close, can be taken as a designated relative position, so when the first jaw 310 and the second jaw 320 start to close, the timing apparatus starts timing.

It should be noted that the timing apparatus obtaining the time parameter after the first jaw 310 and the second jaw 320 are closed to a designated relative position, does not just mean that the timing apparatus starts timing when the first jaw 310 and the second jaw 320 are closed to the designated relative position. For example, the timing can also start to obtain the time parameter after the first jaw 310 and the second jaw 320 has been closed to the designated relative position for a certain time length T.

In some embodiments, when the surgical instrument receives a firing operation of a user, it means that the firing member 420 starts firing the stitching staple. For example, the surgical instrument is provided with a firing switch. When the user presses the firing switch, the pre-squeezing time ends, and the timing apparatus stops or temporarily stops timing.

In some embodiments, the movement rate of the firing member 420 when the firing member 420 starts firing the stitching staple is preset to be within a first preset rate range and in a positive correlation with the pre-squeezing time. The longer the pre-squeezing time is, the better the tissue is squeezed. Therefore, the tissue can be stitched at a faster firing rate. Therefore, in this embodiment, the initial movement rate of the firing member 420 in a positive correlation with the pre-squeezing time, but this positive correlation is also limited to a certain range, that is, it cannot exceed the first preset rate range.

In addition to the pre-squeezing time, a first clamping force of the first jaw 310 and the second jaw 320 on the tissue within the pre-squeezing time can also be obtained, and then the movement rate of the firing member 420 when the firing member 420 starts firing the stitching staple can be determined according to the pre-squeezing time and the first clamping force within the pre-squeezing time. For example, the first jaw 310 and the second jaw 320 are respectively provided with a pressure sensor, and the pressure values which are acquired by the two pressure sensors within the pre-squeezing time are used as the first clamping force by the controller. Of course, a pressure sensor can also be arranged only at the first jaw 310 or the second jaw 320. In some embodiments, the first clamping force obtained is continuous. In other embodiments, the first clamping force can also be obtained at intervals within the pre-squeezing time, that is, the obtained first clamping force is discrete.

In some embodiments, a relationship regarding how the first clamping force within the pre-squeezing time varies with the pre-squeezing time can be obtained according to the pre-squeezing time and the first clamping force within the pre-squeezing time. For example, a relationship curve of the first clamping force which varies with time can be generated according to a continuous or discrete first clamping force. A slope of the relationship curve can indicate a varying rate of the first clamping force. After obtaining the relationship, the movement rate of the firing member 420 when the firing member 420 starts firing the stitching staple(s) can be determined according to the relationship. For example, if a slope of the relationship curve A is K1 and a slope of the relationship curve B is K2, which curves are within a same pre-squeezing time, and K1 is greater than K2; the initial movement rate of the firing member 420, which rate is determined based on the relationship curve A, is greater than the initial movement rate of the firing member 420, which rate is determined based on the relationship curve B. In other embodiments, in addition to the relationship curve, the first clamping force at some designated moments can also be considered in the relationship between the first clamping force and the pre-squeezing time. For example, if a slope of the relationship curve A is K1 and a slope of the relationship curve B is K1, which curves are within a same pre-squeezing time, the first clamping force of the relationship curve A at the end position (that is, when the firing member 420 starts firing the stitching staple) is a, the first clamping force of the relationship curve B at the end position is b, and a is greater than b; then the initial movement rate of the firing member 420, which rate is determined based on the relationship curve A, is less than the initial movement rate of the firing member 420, which rate is determined based on the relationship curve B.

In some embodiments, it is also possible to obtain a statistical value of the first clamping force within the pre-squeezing time, and determine the movement rate when the firing member 420 starts firing the stitching staple according to the statistical value. The statistical value of the first clamping force includes but is not limited to an average value, a maximum value, etc.

After determining the initial movement rate of the firing member 420, the controller controls the drive source with a corresponding drive parameter, so as to enable the drive source to drive, via the transmission mechanism 210, the firing member 420 to move at the determined movement rate. In this process, the first jaw 310 and the second jaw 320 continue to clamp the tissue. In some embodiments, the time parameter also includes a squeezing time of the first jaw 310 and the second jaw 320 to continue to clamp the tissue since they are closed to a designated relative position. The controller can also adjust the movement rate of the firing member 420 while the firing member 420 fires the stitching staple according to the current squeezing time. It should be noted that the squeezing time is a dynamic parameter, which can also be regarded as a sum of the pre-squeezing time and a squeezing time. For example, 10s after the first jaw 310 and the second jaw 320 are closed to the designated position, the operator presses the firing switch, and the pre-squeezing time is 10s. After the firing member 420 starts to move, the timing apparatus continues to time. After moving for 5s, the squeezing time at this time is 15s.

In some embodiments, the movement rate of the firing member 420 while the firing member 420 fires the stitching staple is preset to be within a second preset rate range and in a positive correlation with the squeezing time. For example, with an increase of a current squeezing time, the firing rate also increases linearly or nonlinearly. Alternatively, multiple time gears can be preset, and each time gear has a preset rate value. When the squeezing time exceeds a time gear, the firing rate will be adjusted to a rate value which corresponds to said time gear.

In some embodiments, in addition to the time parameter, the controller can also obtain a second clamping force of the first jaw 310 and the second jaw 320 on the tissue while the firing member 420 fires the stitching staple, and adjust the movement rate of the firing member 420 while the firing member 420 fires the stitching staple, according to the second clamping force. In some embodiments, the movement rate of the firing member 420, while the firing member 420 fires the stitching staple, is preset to be in a negative correlation with the second clamping force, that is, the greater the second clamping force, the lower the movement rate of the firing member 420 will be adjusted to, so as to reduce a stitching rate of the tissue. It can be understood that the second clamping force can be a continuous value obtained while the firing member 420 fires the stitching staple, or a discrete value obtained while the firing member 420 fires the stitching staple.

For example, multiple threshold ranges of clamping force can be preset in advance. While the firing member 420 fires the stitching staple, the controller adjusts the movement rate of the firing member 420 while the firing member 420 fires the stitching staple, according to a threshold range of clamping force into which the second clamping force falls. For example, each threshold range of clamping force corresponds to one movement rate of the firing member 420. If the second clamping force falls into a threshold range of clamping force, the controller adjusts the movement rate of the firing member 420 to the movement rate which corresponds to said threshold range of clamping force. For another example, each threshold range of clamping force corresponds to one rate difference of the firing member 420. If the second clamping force falls into a threshold range of clamping force, the movement rate of the firing member 420 is adjusted based on the initial movement rate of the firing member 420 and the rate difference which corresponds to said threshold range of clamping force.

In some embodiments, in addition to the time parameter, the controller can also obtain a drive resistance of the firing member 420 while the firing member 420 fires the stitching staple, and adjust the movement rate of the firing member 420 while the firing member 420 fires the stitching staple according to the drive resistance. The drive resistance is a resistance to which the transmission mechanism 210 is subjected when pushing the firing member 420 to move. In some embodiments, the movement rate of the firing member 420 while the firing member 420 fires the stitching staple is preset to be in a negative correlation with the drive resistance, that is, the greater the drive resistance is, the lower the movement rate of the firing member 420 will be adjusted to, so as to reduce the stitching rate of the tissue. It can be understood that the drive resistance can be a continuous value obtained while the firing member 420 fires the stitching staple, or a discrete value obtained while the firing member 420 fires the stitching staple.

For example, multiple resistance threshold ranges can be preset in advance. While the firing member 420 fires the stitching staple, the controller adjusts the movement rate of the firing member 420 while the firing member 420 fires the stitching staple, according to a resistance threshold range into which the drive resistance falls. For example, each resistance threshold range corresponds to a movement rate of the firing member 420. If the drive resistance falls into a resistance threshold range, the controller adjusts the movement rate of the firing member 420 to the movement rate which corresponds to said resistance threshold range. For another example, each resistance threshold range corresponds to one rate difference of the firing member 420. If the drive resistance falls into a resistance threshold range, the movement rate of the firing member 420 is adjusted based on the initial movement rate of the firing member 420 and the rate difference which corresponds to said resistance threshold range.

It should be noted that after determining the initial movement rate of the firing member 420, the movement rate of the firing member 420 while the firing member 420 fires the stitching staple can also be adjusted synthetically according to at least two of the squeezing time, the second clamping force and the drive resistance. In addition, the above description of the acquisition methods of the first clamping force, the second clamping force and the drive resistance is only an example. The methods of measuring the first clamping force, the second clamping force and the drive resistance, which methods existed in the past or possible presented in further, fall into the scheme of this disclosure.

Please refer to FIG. 5. The embodiment shown in FIG. 5 also provides a control method for the above surgical instrument, including:
Step S100: determine a movement rate of the firing member 420 when the firing member fires the stitching staple(s) at least according to a time parameter which is related to squeezing the tissue when the first jaw 310 and the second jaw 320 are closed, so as to control the firing member 420 to fire the stitching staple(s) at the movement rate determined.

In some embodiments, in step S100, control the movement rate of the firing member 420 in two dimensions according to the time parameter. The movement rate in a first dimension is the movement rate when the firing member 420 starts firing the stitching staple, that is, an initial movement rate of the firing member 420 at a start position. The movement rate in the second dimension is the movement rate of the firing member 420 while the firing member 420 fires the stitching staple. That is to say, continuously adjust the movement rate of the firing member 420 from a start position to an end position.

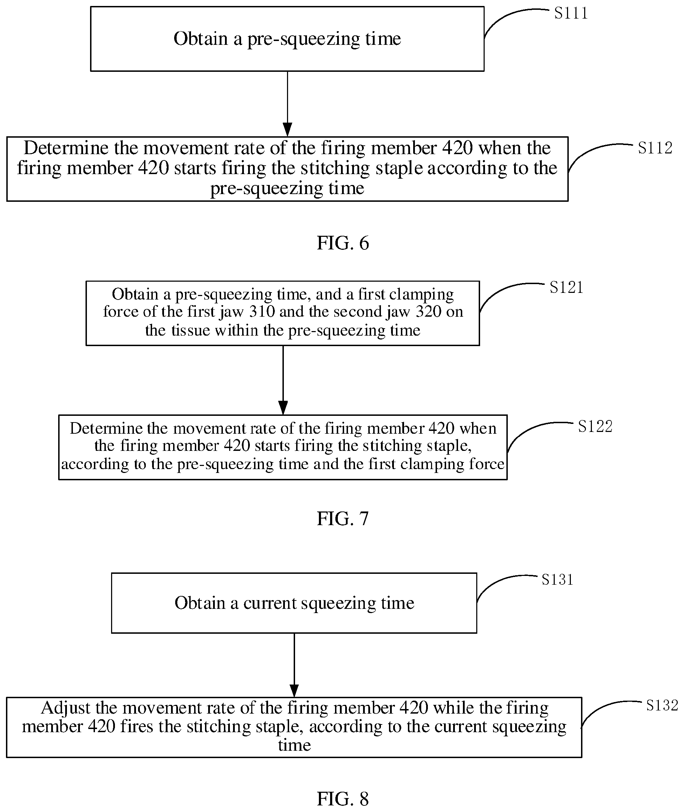

In some embodiments, as shown in FIG. 6, the step S100 specifically includes following steps.

Step S111: obtain a pre-squeezing time.

The pre-squeezing time is a time length which is timed from a time point when the first jaw 310 and the second jaw 320 are closed to a designated relative position to a time point when the firing member 420 starts firing the stitching staple. The designated relative position can be customized by a user according to a situation, or can also be a relative position that satisfies a preset condition, including but not limited to following positions.

The designated relative position can be a position where the first jaw 310 and the second jaw 320 start to clamp the tissue. For example, both the first jaw 310 and the second jaw 320 are respectively provided with a pressure sensor. When a certain value appears on both pressure sensors, it means that both the first jaw 310 and the second jaw 320 are in contact with the tissue.

The designated relative position can be a designated position to where the first jaw 310 and the second jaw 320 clamp the tissue. For example, the first jaw 310 and the second jaw 320 are respectively provided with a pressure sensor. When a measurement result of the pressure sensor exceeds a preset pressure threshold, it means that the first jaw 310 and the second jaw 320 clamp the tissue to a designated position.

The designated relative position can be a position at which the first jaw 310 and the second jaw 320 are closed to a preset distance from each other, or the first jaw 310 and the second jaw 320 are closed to a preset angle. Even in some embodiments, a position, where the first jaw 310 and the second jaw 320 start to close, can be taken as a designated relative position.

In some embodiments, when the surgical instrument receives a firing operation of a user, it means that the firing member 420 starts firing the stitching staple. For example, the surgical instrument is provided with a firing switch. When the user presses the firing switch, the pre-squeezing time ends.

Step S112: determine the movement rate of the firing member 420 when the firing member 420 starts firing the stitching staple according to the pre-squeezing time.

In some embodiments, the movement rate of the firing member 420 when the firing member 420 starts firing the stitching staple is preset to be within a first preset rate range and in a positive correlation with the pre-squeezing time. The longer the pre-squeezing time is, the better the tissue is squeezed. Therefore, the tissue can be stitched at a faster firing rate. Therefore, in this embodiment, the initial movement rate of the firing member 420 in a positive correlation with the pre-squeezing time, but this positive correlation is also limited to a certain range, that is, it cannot exceed the first preset rate range.

In some embodiments, as shown in FIG. 7, the step S100 specifically includes following steps.

Step S121: obtain a pre-squeezing time, and a first clamping force of the first jaw 310 and the second jaw 320 on the tissue within the pre-squeezing time.

Wherein, the meaning of pre-squeezing time has been described above, and will not be repeated here. In some embodiments, the first clamping force obtained is continuous. In other embodiments, the first clamping force can also be obtained at intervals within the pre-squeezing time, that is, the obtained first clamping force is discrete.

Step S122: determine the movement rate of the firing member 420 when the firing member 420 starts firing the stitching staple, according to the pre-squeezing time and the first clamping force within the pre-squeezing time.

In some embodiments, a relationship regarding how the first clamping force within the pre-squeezing time varies with the pre-squeezing time can be obtained according to the pre-squeezing time and the first clamping force within the pre-squeezing time. For example, a relationship curve of the first clamping force which varies with time can be generated according to a continuous or discrete first clamping force. A slope of the relationship curve can indicate a varying rate of the first clamping force. After obtaining the relationship, the movement rate of the firing member 420 when the firing member 420 starts firing the stitching staple(s) can be determined according to the relationship. For example, if a slope of the relationship curve A is K1 and a slope of the relationship curve B is K2, which curves are within a same pre-squeezing time, and K1 is greater than K2; the initial movement rate of the firing member 420, which rate is determined based on the relationship curve A, is greater than the initial movement rate of the firing member 420, which rate is determined based on the relationship curve B. In other embodiments, in addition to the relationship curve, the first clamping force at some designated moments can also be considered in the relationship between the first clamping force and the pre-squeezing time. For example, if a slope of the relationship curve A is K1 and a slope of the relationship curve B is K1, which curves are within a same pre-squeezing time, the first clamping force of the relationship curve A at the end position (that is, when the firing member 420 starts firing the stitching staple) is a, the first clamping force of the relationship curve B at the end position is b, and a is greater than b; then the initial movement rate of the firing member 420, which rate is determined based on the relationship curve A, is less than the initial movement rate of the firing member 420, which rate is determined based on the relationship curve B.

In some embodiments, it is also possible to obtain a statistical value of the first clamping force within the pre-squeezing time, and determine the movement rate when the firing member 420 starts firing the stitching staple according to the statistical value. The statistical value of the first clamping force includes but is not limited to an average value, a maximum value, etc.

In some embodiments, after the firing member starts firing the stitching staple, as shown in FIG. 8, the step S100 further includes following steps.

Step S131: obtain a current squeezing time.

The squeezing time is a time that the first jaw 310 and the second jaw 320 continue to clamp the tissue since they are closed to the designated relative position. It should be noted that the squeezing time is a dynamic parameter, which can also be regarded as a sum of the pre-squeezing time and the squeezing time. For example, 10s after the first jaw 310 and the second jaw 320 are closed to the designated position, the operator presses the firing switch, and the pre-squeezing time is 10s. After the firing member 420 starts to move, the timing apparatus continues to time. After moving for 5s, the squeezing time at this time is 15s.

Step S132: adjust the movement rate of the firing member 420 while the firing member 420 fires the stitching staple, according to the current squeezing time.

In some embodiments, the movement rate of the firing member 420 while the firing member 420 fires the stitching staple is preset to be within a second preset rate range and in a positive correlation with the squeezing time. For example, with an increase of a current squeezing time, the firing rate also increases linearly or nonlinearly. Alternatively, multiple time gears can be preset, and each time gear has a preset rate value. When the squeezing time exceeds a time gear, the firing rate will be adjusted to a rate value which corresponds to said time gear.

In some embodiments, in addition to the time parameter, it is also possible to obtain a second clamping force of the first jaw 310 and the second jaw 320 on the tissue while the firing member 420 fires the stitching staple, and adjust the movement rate of the firing member 420 while the firing member 420 fires the stitching staple, according to the second clamping force. In some embodiments, the movement rate of the firing member 420, while the firing member 420 fires the stitching staple, is preset to be in a negative correlation with the second clamping force, that is, the greater the second clamping force, the lower the movement rate of the firing member 420 will be adjusted to, so as to reduce a stitching rate of the tissue. It can be understood that the second clamping force can be a continuous value obtained while the firing member 420 fires the stitching staple, or a discrete value obtained while the firing member 420 fires the stitching staple.

For example, multiple threshold ranges of clamping force can be preset in advance. While the firing member 420 fires the stitching staple, adjust the movement rate of the firing member 420 while the firing member 420 fires the stitching staple, according to a threshold range of clamping force into which the second clamping force falls. For example, each threshold range of clamping force corresponds to one movement rate of the firing member 420. If the second clamping force falls into a threshold range of clamping force, adjust the movement rate of the firing member 420 to the movement rate which corresponds to said threshold range of clamping force. For another example, each threshold range of clamping force corresponds to one rate difference of the firing member 420. If the second clamping force falls into a threshold range of clamping force, the movement rate of the firing member 420 is adjusted based on the initial movement rate of the firing member 420 and the rate difference which corresponds to said threshold range of clamping force.

In some embodiments, in addition to the time parameter, it is also possible to obtain a drive resistance of the firing member 420 while the firing member 420 fires the stitching staple, and adjust the movement rate of the firing member 420 while the firing member 420 fires the stitching staple according to the drive resistance. The drive resistance is a resistance to which the transmission mechanism 210 is subjected when pushing the firing member 420 to move. In some embodiments, the movement rate of the firing member 420 while the firing member 420 fires the stitching staple is preset to be in a negative correlation with the drive resistance, that is, the greater the drive resistance is, the lower the movement rate of the firing member 420 will be adjusted to, so as to reduce the stitching rate of the tissue. It can be understood that the drive resistance can be a continuous value obtained while the firing member 420 fires the stitching staple, or a discrete value obtained while the firing member 420 fires the stitching staple.

For example, multiple resistance threshold ranges can be preset in advance. While the firing member 420 fires the stitching staple, adjust the movement rate of the firing member 420 while the firing member 420 fires the stitching staple, according to a resistance threshold range into which the drive resistance falls. For example, each resistance threshold range corresponds to a movement rate of the firing member 420. If the drive resistance falls into a resistance threshold range, adjust the movement rate of the firing member 420 to the movement rate which corresponds to said resistance threshold range. For another example, each resistance threshold range corresponds to one rate difference of the firing member 420. If the drive resistance falls into a resistance threshold range, the movement rate of the firing member 420 is adjusted based on the initial movement rate of the firing member 420 and the rate difference which corresponds to said resistance threshold range.

The description has been made with reference to various exemplary embodiments herein. However, those skilled in the art recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disk, floppy disk, etc.), optical storage devices (CD-ROM, DVD, Blu Ray disk, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments are included in the scope of this disclosure.

The foregoing specific description has been described with reference to various embodiments. However, those skilled in the art recognize that various modifications and changes can be made without departing from the scope of the present disclosure. Therefore, consideration of the present disclosure is in an illustrative rather than a restrictive sense, and all such modifications are included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "including", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. A surgical instrument, **characterized in that**, comprising
a handle assembly;
a barrel assembly, wherein a proximal end of the barrel assembly is connected with the handle assembly, and the barrel assembly comprises a transmission mechanism;
an end effector assembly, wherein a distal end of the barrel assembly is connected with the end effector assembly, and the end effector assembly comprises a first jaw and a second jaw for clamping a tissue;
a staple cartridge assembly, which is arranged between the first jaw and the second jaw, and comprises a firing member which is connected with the transmission mechanism;
a drive source, which is capable of driving, via the transmission mechanism, the firing member to move, wherein the firing member is configured to fire one or more stitching staples to stitch the tissue while moving;
a controller, which is at least configured to determine a movement rate of the firing member when the firing member fires the stitching staple(s), according to a time parameter which is related to squeezing the tissue when the first jaw and the second jaw are closed, so as to control the firing member to fire the stitching staple(s) at the movement rate determined.

2. The surgical instrument according to claim 1, **characterized in that**, in order to determine a movement rate of the firing member when the firing member fires the stitching staple(s), according to a time parameter which is related to squeezing the tissue when the first jaw and the second jaw are closed, the controller is specifically configured to:
determine, at least according to the time parameter, the movement rate of the firing member when the firing member starts firing the stitching staple(s).

3. The surgical instrument according to claim 2, **characterized in that**, the time parameter comprises a pre-squeezing time, which is from a time point when the first jaw and the second jaw are closed to a designated relative position to a time point when the firing member starts firing the stitching staple(s);
in order to determine, at least according to the time parameter, the movement rate of the firing member when the firing member starts firing the stitching staple(s), the controller is specifically configured to:
determine, at least according to the pre-squeezing time, the movement rate of the firing member when the firing member starts firing the stitching staple(s).

4. The surgical instrument according to claim 3, **characterized in that**, the movement rate of the firing member, when the firing member starts firing the stitching staple(s), is preset to be within a first preset rate range and in a positive correlation with the pre-squeezing time.

5. The surgical instrument according to claim 3, **characterized in that**, in order to determine, at least according to the pre-squeezing time, the movement rate of the firing member when the firing member starts firing the stitching staple(s), the controller is specifically configured to:
obtain a first clamping force of the first jaw and the second jaw on the tissue within the pre-squeezing time; and
determine, according to the pre-squeezing time and the first clamping force within the pre-squeezing time, the movement rate of the firing member when the firing member starts firing the stitching staple(s).

6. The surgical instrument according to claim 5, **characterized in that**, in order to determine, according to the pre-squeezing time and the first clamping force within the pre-squeezing time, the movement rate of the firing member when the firing member starts firing the stitching staple(s), the controller is specifically configured to:
obtain a relationship regarding how the first clamping force within the pre-squeezing time varies with the pre-squeezing time, and determine, according to the relationship, the movement rate of the firing member when the firing member starts firing the stitching staple(s); or
obtain a statistical value of the first clamping force within the pre-squeezing time, and determine, according to the statistical value, the movement rate of the firing member when the firing member starts firing the stitching staple(s).

7. The surgical instrument according to any one of claims 1~6, **characterized in that**, in order to determine a movement rate of the firing member when the firing member fires the stitching staple(s), according to a time parameter which is related to squeezing the tissue when the first jaw and the second jaw are closed, the controller is specifically configured to:
adjust, according to the time parameter, the movement rate of the firing member while the firing member fires the stitching staple(s).

8. The surgical instrument according to claim 7, **characterized in that**, the first jaw and the second jaw are configured to continue to clamp the tissue when the firing member fires the stitching staple(s); the time parameter comprises a squeezing time for the first jaw and the second jaw to continue to clamp the tissue since when the first jaw and the second jaw are closed to a designated relative position;
in order to adjust, according to the time parameter, the movement rate of the firing member while the firing member fires the stitching staple(s), the controller is specifically configured to:
adjust, according to the squeezing time, the movement rate of the firing member while the firing member fires the stitching staple(s).

9. The surgical instrument according to claim 8, **characterized in that**, the movement rate of the firing member, while the firing member fires the stitching staple(s), is preset to be within a second preset rate range and in a positive correlation with the squeezing time.

10. The surgical instrument according to any one of claims 1~9, **characterized in that**, the controller is further configured to:
obtain a second clamping force of the first jaw and the second jaw on the tissue while the firing member fires the stitching staple(s), and/or obtain a drive resistance while the firing member fires the stitching staple(s); and
adjust, according to the second clamping force and/or the drive resistance, the movement rate of the firing member while the firing member fires the stitching staple(s).

11. The surgical instrument according to claim 10, **characterized in that**, the movement rate of the firing member, while the firing member fires the stitching staple(s), is preset to be in a negative correlation with the second clamping force and/or the drive resistance.

12. The surgical instrument according to any one of claims 1~11, **characterized in that**, further comprising a timing apparatus that starts timing after the first jaw and the second jaw are closed to a designated relative position, so as to obtain the time parameter.

13. A control method for a surgical instrument, **characterized in that**, the surgical instrument comprises:
a handle assembly;
a barrel assembly, wherein a proximal end of the barrel assembly is connected with the handle assembly, and the barrel assembly comprises a transmission mechanism;
an end effector assembly, wherein a distal end of the barrel assembly is connected with the end effector assembly, and the end effector assembly comprises a first jaw and a second jaw for clamping a tissue;
a staple cartridge assembly, which is arranged between the first jaw and the second jaw, and comprises a firing member which is connected with the transmission mechanism;
a drive source, which is capable of driving, via the transmission mechanism, the firing member to move, wherein the firing member is configured to fire one or more stitching staples to stitch the tissue while moving;
wherein the method comprises:
at least determining a movement rate of the firing member when the firing member fires the stitching staple(s), according to a time parameter which is related to squeezing the tissue when the first jaw and the second jaw are closed, so as to control the firing member to fire the stitching staple(s) at the movement rate determined.

14. The method according to claim 13, **characterized in that**, determining a movement rate of the firing member when the firing member fires the stitching staple(s), according to a time parameter which is related to squeezing the tissue when the first jaw and the second jaw are closed, comprises:
determining, at least according to the time parameter, the movement rate of the firing member when the firing member starts firing the stitching staple(s).

15. The method according to claim 14, **characterized in that**, the time parameter comprises a pre-squeezing time, which is from a time point when the first jaw and the second jaw are closed to a designated relative position to a time point when the firing member starts firing the stitching staple(s);
determining, at least according to the time parameter, the movement rate of the firing member when the firing member starts firing the stitching staple(s), comprises:
determining, at least according to the pre-squeezing time, the movement rate of the firing member when the firing member starts firing the stitching staple(s).

16. The method according to claim 15, **characterized in that**, the movement rate of the firing member, when the firing member starts firing the stitching staple(s), is preset to be within a first preset rate range and in a positive correlation with the pre-squeezing time.

17. The method according to claim 16, **characterized in that**, determining, at least according to the pre-squeezing time, the movement rate of the firing member when the firing member starts firing the stitching staple(s), comprises:
obtaining a first clamping force of the first jaw and the second jaw on the tissue within the pre-squeezing time; and
determining, according to the pre-squeezing time and the first clamping force within the pre-squeezing time, the movement rate of the firing member when the firing member starts firing the stitching staple(s).

18. The method according to claim 17, **characterized in that**, determining, according to the pre-squeezing time and the first clamping force within the pre-squeezing time, the movement rate of the firing member when the firing member starts firing the stitching staple(s), comprises:
obtaining a relationship regarding how the first clamping force within the pre-squeezing time varies with the pre-squeezing time, and determining, according to the relationship, the movement rate of the firing member when the firing member starts firing the stitching staple(s); or
obtaining a statistical value of the first clamping force within the pre-squeezing time, and determining, according to the statistical value, the movement rate of the firing member when the firing member starts firing the stitching staple(s).

19. The method according to any one of claims 13~18, **characterized in that**, determining a movement rate of the firing member when the firing member fires the stitching staple(s), according to a time parameter which is related to squeezing the tissue when the first jaw and the second jaw are closed, comprises:
adjusting, according to the time parameter, the movement rate of the firing member while the firing member fires the stitching staple(s).

20. The method according to claim 19, **characterized in that**, the first jaw and the second jaw are configured to continue to clamp the tissue when the firing member fires the stitching staple(s); the time parameter comprises a squeezing time for the first jaw and the second jaw to continue to clamp the tissue since when the first jaw and the second jaw are closed to a designated relative position;
adjusting, according to the time parameter, the movement rate of the firing member while the firing member fires the stitching staple(s), comprises:
adjusting, according to the squeezing time, the movement rate of the firing member while the firing member fires the stitching staple(s).

21. The method according to claim 20, **characterized in that**, the movement rate of the firing member, while the firing member fires the stitching staple(s), is preset to be within a second preset rate range and in a positive correlation with the squeezing time.

22. The method according to any one of claims 13~21, **characterized in that**, further comprising:
obtaining a second clamping force of the first jaw and the second jaw on the tissue while the firing member fires the stitching staple(s), and/or obtaining a drive resistance while the firing member fires the stitching staple(s); and
adjusting, according to the second clamping force and/or the drive resistance, the movement rate of the firing member while the firing member fires the stitching staple(s).

23. The method according to claim 22, **characterized in that**, the movement rate of the firing member, while the firing member fires the stitching staple(s), is preset to be in a negative correlation with the second clamping force and/or the drive resistance.

24. A computer-readable storage medium on which a program is stored, **characterized in that**, the program is capable of being executed by a processor, so as to implement the method according to any one of claims 13~23.
